# EUROPÄISCHE PATENTSCHRIFT

(11) **EP 3 066 655 B1**
(45) Veröffentlichungstag und Bekanntmachung des Hinweises auf die Patenterteilung: **03.06.2020**
(21) Anmeldenummer: 14795632.0
(22) Anmeldetag: 07.11.2014
(51) Int. Cl.: G09B 19/00, A61B 5/11

(54) **VORRICHTUNG UND VERFAHREN ZUM AUTOMATISCHEN BEWERTEN EINES VERLAUFES EINER TRAININGSÜBUNG**
APPARATUS AND METHOD FOR THE AUTOMATIC EVALUATION OF THE COURSE OF A TRAINING SESSION
APPAREIL ET PROCÉDÉ D'EVALUATION AUTOMATIQUE DU DÉROULEMENT D'UNE SESSION D'ENTRAÎNEMENT

(30) Priorität: 08.11.2013 DE 102013112317
(43) Veröffentlichungstag der Anmeldung: 14.09.2016
(73) Patentinhaber: Fraunhofer-Gesellschaft zur Förderung der angewandten Forschung e.V., 80686 München (DE)
(72) Erfinder: EDELHÄUSSER, Thorsten, 91077 Neuenkirchen a. Brand (DE); WITT, Nicolas, 90459 Nürnberg (DE); VÖLKER, Matthias, 91093 Hessdorf (DE); VOLL, David, 90552 Röthenbach a. d. Pegnitz (DE); OTTO, Stephan, 90562 Heroldsberg (DE); BRETZ, Ingmar, 93128 Regenstauf (DE); FRANKE, Norbert, 91058 Erlangen (DE)
(74) Vertreter: 2SPL Patentanwälte PartG mbB
(86) Internationale Anmeldenummer: PCT/EP2014/074041
(87) Internationale Veröffentlichungsnummer: WO 2015/067753

(56) Entgegenhaltungen:
- EP-A2- 2 189 191
- WO-A1-2013/041124
- WO-A1-2013/064172
- WO-A1-2013/064174
- WO-A1-2013/126655
- US-A1- 2003 087 220
- US-A1- 2008 258 921
- US-A1- 2009 210 078
- US-A1- 2011 169 959
- US-A1- 2012 053 828
- US-B1- 6 837 827

## Beschreibung

Ausführungsbeispiele der vorliegenden Erfindung befassen sich mit einer Vorrichtung und einem Verfahren zum automatischen Bewerten eines Verlaufs einer Trainingsübung, insbesondere im Sport.

Das Bewerten einer Trainingsübung im Sport kann hilfreich sein, um einen direkten und objektiven Vergleich zwischen den Leistungen einzelner Teilnehmer an der Trainingsübung zu erhalten. Ferner kann die Bewertung des Verlaufs der Trainingsübung dabei helfen zu erkennen, dass es sinnvoll ist, die Trainingsübung selbst zu verändern. Häufig wird hierzu die Videoanalyse verwendet, d.h. die an der Trainingsübung beteiligten Objekte wie beispielsweise Sportler oder Spielgeräte wie Bälle oder dergleichen werden während des Übungsverlaufs mittels Videoaufzeichnungsgeräten gefilmt. Der Verlauf der Übung bzw. des Trainings wird beispielsweise vom Trainer durch Sichten des Videomaterials bewertet. Dies ist äußerst zeitaufwändig und darüber hinaus mit einer hohen Latenz zwischen dem tatsächlichen Abhalten der Übung und dem Erhalt der Ergebnisse verbunden. Ferner ist bei der Bewertung immer ein subjektiver Aspekt vorhanden, der aus der Bewertung der Szenen durch den Trainer bzw. des Blickwinkels der Videokameras entsteht. Dies kann dazu führen, dass der direkte Vergleich zwischen zwei an der Trainingsübung beteiligten Sportlern oder Objekten auf objektive Art und Weise nur schwierig möglich ist.

Ferner sind aus den Druckschriften US 6 837 827 Bl, WO 2013/064174 A1, US 2003/087220 A1, US 2009/210078 A1, EP 2 189 191 A2, WO 2013/064172 A1, WO 2013/126655 A1, US 2012/053828 A1, US 2011/169959 A1, US 2008/258921 A1 und WO 2013/041124 A1 diverse Ansätze zur Überwachung und Auswertung von Übungen bzw. Bewegungsabläufen von Sportlern bekannt.

Es besteht das Bedürfnis, die Bewertung eines Verlaufs einer Trainingsübung zu verbessern. Ausführungsbeispiele der vorliegenden Erfindung ermöglichen dies durch ein Verfahren gemäß dem unabhängigen Anspruch 1, ein System gemäß dem unabhängigen Anspruch 8 bzw. ein Programm gemäß dem unabhängigen Anspruch 10.

Ein Ausführungsbeispiel eines Verfahrens zum automatischen Bewerten eines Verlaufs einer Trainingsübung umfasst ein Bereitstellen einer Mehrzahl von Ortsinformationen für zumindest ein an der Trainingsübung teilnehmendes bewegliches Objekt. Das Bereitstellen der Mehrzahl von Ortsinformationen umfasst das Bestimmen der Position des beweglichen Objektes innerhalb des Übungsareals mittels eines Positionsverfolgungssystems zu mehreren aufeinanderfolgenden Zeitpunkten. Weiterhin umfasst das Verfahren ein Bereitstellen zumindest einer weiteren Ortsinformationen unter Verwendung einer graphischen Benutzerschnittstelle. Die graphische Benutzerschnittstelle umfasst eine graphische Repräsentation des Übungsareals sowie eine graphische Repräsentation eines an der Trainingsübung beteiligten Übungsobjektes in einer grafischen Anzeige. Die weitere Ortsinformation gibt die Position des an der Trainingsübung beteiligten Übungsobjektes innerhalb des Übungsareals an, wobei das Übungsobjekt prinzipiell beweglich ist, eine Bewegung des Übungsobjekts aber nicht Ziel der Trainingsübung ist. Ferner wird zumindest ein vorbestimmtes Übungsziel bereitgestellt und die Mehrzahl von Ortsinformationen für das bewegliche Objekt werden mit dem vorbestimmten Übungsziel verglichen. Das Bereitstellen des Übungsziels umfasst das Definieren einer gewünschten Interaktion zwischen dem beweglichen Objekt und dem an der Trainingsübung beteiligten Übungsobjekt. Der Verlauf der Trainingsübung wird basierend auf dem Ergebnis dieses Vergleiches bewertet. Das Bereitstellen des zumindest einen vorbestimmten Übungsziels umfasst ein Auswählen zumindest einer verwendeten Auswertelogik für die Ortsinformationen aus einer Mehrzahl zur Verfügung stehender Auswertelogiken. Das Bereitstellen der weiteren Ortsinformation umfasst ein Vergleichen der Position der graphischen Repräsentation des Übungsobjektes innerhalb der graphischen Repräsentation des Übungsareals mit einer mittels des Positionsverfolgungssystems bestimmten Position des Übungsobjektes innerhalb des Übungsareals. Das Verfahren umfasst ferner ein Bereitstellen eines akustischen, visuellen oder haptischen Signals durch das Positionsverfolgungssystem. Das Signal umfasst eine Information über eine Abweichung zwischen der mittels des Positionsverfolgungssystems bestimmten Position des Übungsobjektes innerhalb des Übungsareals und der Position der graphischen Repräsentation des Übungsobjektes innerhalb der graphischen Repräsentation des Übungsareals, so dass die Position des Übungsobjektes innerhalb des Übungsareals beim Aufbau der Trainingsübung derart verändert werden kann, dass die mittels des Positionsverfolgungssystems bestimmte Position des Übungsobjektes innerhalb des Übungsareals der Position der graphischen Repräsentation des Übungsobjektes innerhalb der graphischen Repräsentation des Übungsareals entspricht. Das Bereitstellen der Mehrzahl von Ortsinformationen für ein bewegliches Objekt in Verbindung mit dem Bereitstellen bzw. dem vorherigen Definieren zumindest eines vorbestimmten Übungsziels ermöglicht es, den Verlauf einer Trainingsübung automatisiert und objektiv zu bewerten. Beispielsweise kann so auch eine qualitative Aussage über einen Erfolg abgeleitet werden.

Ferner kann die Bewertung zeitnah erfolgen. Gemäß einigen Ausführungsbeispielen kann die Bewertung in Echtzeit oder mit sehr geringer Latenz erfolgen, sodass gegebenenfalls entschieden werden kann, dass eine Wiederholung der Trainingsübung erforderlich ist, was herkömmlich kaum möglich ist. So kann gegebenenfalls der Aufwand für das zeitlich versetzte erneute Aufbauen der Trainingsübung gespart werden. Aufgrund der objektiven Auswertung können jedoch auch zeitlich beabstandete identische Trainingsübungen mit denselben Maßstäben bewertet werden, was auch über lange Zeiträume einen Vergleich von an den Trainingsübungen teilnehmenden Objekten ermöglicht. An der Trainingsübung teilnehmende bewegliche Objekte können beispielsweise Sportler sein, deren Position innerhalb eines vorbestimmten Übungsareals mittels eines Positionsverfolgungssystems während des Durchführens der Trainingsübung aufgezeichnet bzw. bestimmt wurde. Allgemein gesprochen kann ein an der Trainingsübung teilnehmendes bewegliches Objekt jedoch auch ein beliebiges anderes Objekt sein, beispielsweise ein Sportgegenstand wie ein Ball, ein geworfener Speer, ein geworfener Diskus oder ein Stab eines Stabhochspringers bzw. die Latte, die von einem Hochspringer zu überwinden ist.
Erfindungsgemäß umfasst das Bereitstellen des zumindest einen vorbestimmten Übungsziels ein Auswählen zumindest einer verwendeten Auswertelogik für die Ortsinformationen aus einer Mehrzahl zur Verfügung stehender Auswertelogiken. Durch die Möglichkeit, unterschiedliche Auswertelogiken zu verwenden, können gemäß den Ausführungsbeispielen der Erfindung beliebige Fragestellungen für beliebige Sportarten oder Anwendungsfälle beantwortet werden. Das Auswählen einer Auswertelogik umfasst je nach Anwendungsfall das bloße Auswählen einer vordefinierten Auswertelogik, das Anpassen oder Verändern einer vordefinierten Auswertelogik und das definieren einer neuen Auswertelogik, was es ermöglichen kann, beliebigen Fragestellungen nachzugehen.

Gemäß der verwendeten Auswertelogik wird gemäß einigen Ausführungsbeispielen ein taktischer Parameter oder ein technischer Parameter für eine Sportart bestimmt wird. Gemäß diesen Ausführungsbeispielen können somit beispielsweise Trainingsübungen bewertet werden, die es zum Ziel haben, taktische Verhaltensweisen zu trainieren. Die geeignete Auswertelogik ermöglicht somit die Beantwortung von komplizierteren Fragestellungen, die über Informationen, die mit dem beobachteten Ort direkt korrelieren, weit hinausgehen können. Gemäß einigen Ausführungsbeispielen kann mittels der verwendeten Auswertelogik basierend auf den Ortsinformationen jedoch auch oder zusätzlich ein athletischer Parameter für zumindest eine an der Trainingsübung beteiligte Person bestimmt werden. Dabei versteht es sich von selbst, dass gemäß den Ausführungsbeispielen der Erfindung mehrere Fragestellungen gleichzeitig nachgegangen werden kann, wozu während des Bereitstellens des Übungsziels beispielsweise mehrere zu verwendende Auswertelogiken ausgewählt oder definiert werden können.

Erfindungsgemäß zeigt eine Ortsinformation für das Objekt jeweils eine Position des Objektes innerhalb des Übungsareals an. Ein relativer Bezug der Position auf ein Übungsareal kann es ermöglichen, ein und dieselbe Trainingsübung an unterschiedlichen physikalischen Orten abzuhalten, wenn die Koordinaten jeweils bezogen auf das Übungsareal und nicht in einem absoluten Koordinatensystem angegeben werden. Das Koordinatensystem bzw. dessen Darstellung selbst kann beliebig sein, beispielsweise können die Positionen der an den Trainingsübungen beteiligten beweglichen und unbeweglichen Objekte in kartesischen Koordinaten, in Polarkoordinaten oder in geografischen Koordinaten angegeben werden. Auch die Anzahl der Dimensionen ist nicht festgelegt. Je nach Ausführungsbeispiel können ein-, zwei-, oder drei-dimensionale Koordinaten verwendet werden.

Erfindungsgemäß wird die Mehrzahl von Ortsinformationen von einem Positionsverfolgungssystem bereitgestellt, das die Position des beweglichen Objektes innerhalb des Übungsareals zu mehreren aufeinanderfolgenden Zeitpunkten erfasst. Ein solches Tracking-System liefert beispielsweise Positionen für Sportler, Übungsmaterial bzw. Übungsobjekten und andere Abgrenzungen des Übungsareals. Die Positionen der Objekte können dabei auf beliebige Art und Weise gewonnen werden, beispielsweise mittels eines videobasierten, eines funkbasierten oder eines akustischen Ortungs- bzw. Positionsverfolgungssystems.

Erfindungsgemäß wir auch ein ortsfestes Übungsgerät bei der Bewertung des Erfolgs der Trainingsübung berücksichtigt. Dazu wird zumindest eine weitere Ortsinformation für ein Übungsobjekt bereitgestellt. Eine Ortsinformation für ein Übungsobjekt korrespondiert dabei zu einem Objekt, das sich nicht aus eigener Kraft bewegen kann. Solche Objekte bzw. ortsfestes oder unbewegliches Übungsmaterial können beispielsweise Hütchen, Stangen oder Aufsteller sein, sowie Linien, die Spielfelder oder vorgegebene Bewegungsrichtungen angeben bzw. begrenzen. Übungsobjekte in diesem Sinne sind somit beispielsweise reale Objekte, die zwar prinzipiell beweglich sein können, wie beispielsweise Hütchen oder dergleichen, deren Bewegung jedoch nur durch Interaktion mit einem Sportler oder einem anderen beweglichen Objekt erfolgen kann. Mit anderen Worten können Übungsobjekte solche Objekte sein, deren mögliche Bewegung nicht ein Ziel der Übung ist. In diesem Kontext können Übungsobjekte nachfolgend auch als unbewegliche Objekte bezeichnet werden.

Erfindungsgemäß wird die Ortsinformation bzw. die Position eines an der Trainingsübung beteiligten unbeweglichen Objektes von einem Benutzer direkt vorgegeben. Dies erfolgt durch Eingabe der zugeordneten Koordinaten mittels einer grafischen Benutzeroberfläche, die eine grafische Darstellung des Übungsareals sowie der an der Trainingsübung beteiligten Objekte umfasst. Entsprechend kann die Position des unbeweglichen Objektes durch Bewegen einer grafischen Repräsentation des Objektes innerhalb der grafischen Darstellung des Übungsareals bis zu deren gewünschter Position erfolgen.

Erfindungsgemäß umfasst das Bereitstellen des vorbestimmten Übungsziels auch eine Definition von Parametern, die für das bewegliche Objekt basierend auf den Ortsinformationen zunächst bestimmt werden sollen, um den Erfolg der Trainingsübung zu bewerten. Solche Parameter können beispielsweise die Geschwindigkeit des Objektes, die Geschwindigkeit einer Richtungsänderung des Objektes, eine zurückgelegte Wegstrecke innerhalb einer vorbestimmten Zeiteinheit, eine Anzahl von Richtungsänderungen pro Zeiteinheit oder dergleichen sein.

Einige Ausführungsbeispiele umfassen ferner ein System zum Bewerten eines Erfolges eines Verlaufs einer Trainingsübung, das ein Positionserfassungssystem aufweist, welches ausgebildet ist, eine Position eines beweglichen Objektes innerhalb eines Übungsareals zu mehreren aufeinanderfolgenden Zeitpunkten zu bestimmen, um eine Mehrzahl an Ortsinformationen für das bewegliche Objekt bereitzustellen, und eine Position des Übungsobjektes innerhalb des Übungsareals zu bestimmen. Das Übungsobjekt ist prinzipiell beweglich, eine Bewegung des Übungsobjekts ist aber nicht Ziel der Trainingsübung. Das System umfasst ferner einen Analysator mit einer Eingangsschnittstelle, die mit dem Positionserfassungssystem zum Empfang der Mehrzahl an Ortsinformationen und der durch das Positionserfassungssystem bestimmten Position des Übungsobjektes verbunden ist. Der Analysator umfasst ferner eine graphische Benutzerschnittstelle, die eine graphischen Repräsentation des Übungsareals sowie eine graphische Repräsentation des Übungsobjektes in einer grafischen Anzeige umfasst, und mittels derer eine weitere Ortsinformation bereitgestellt wird. Die weitere Ortsinformation gibt die Position des an der Trainingsübung beteiligten Übungsobjektes innerhalb des Übungsareals an. Der Analysator umfasst weiterhin einen Speicher, der ausgebildet ist, um zumindest ein vorbestimmtes Übungsziel bereitzustellen. Das Übungsziel definiert eine gewünschte Interaktion zwischen dem beweglichen Objekt und dem an der Trainingsübung beteiligten Übungsobjekt. Das zumindest eine vorbestimmte Übungsziel umfasst zumindest eine aus einer Mehrzahl zur Verfügung stehender Auswertelogiken ausgewählte Auswertelogik für die Ortsinformationen. Zudem umfasst der Analysator einen Vergleicher, der ausgebildet ist, um die Mehrzahl von Ortsinformationen mit dem vorbestimmten Übungsziel zu vergleichen, und einen Bewerter, der ausgebildet ist, um den Verlauf der Trainingsübung basierend auf dem Ergebnis des Vergleiches zu bewerten. Das Positionsverfolgungssystem ist ferner ausgebildet, ein akustisches, visuelles oder haptisches Signal, das eine Information über eine Abweichung zwischen der mittels des Positionsverfolgungssystems bestimmten Position des Übungsobjektes innerhalb des Übungsareals und der Position der graphischen Repräsentation des Übungsobjektes innerhalb der graphischen Repräsentation des Übungsareals umfasst, bereitzustellen, so dass die Position des Übungsobjektes innerhalb des Übungsareals beim Aufbau der Trainingsübung derart verändert werden kann, dass die mittels des Positionsverfolgungssystems bestimmte Position des Übungsobjektes innerhalb des Übungsareals der Position der graphischen Repräsentation des Übungsobjektes innerhalb der graphischen Repräsentation des Übungsareals entspricht.

Bevorzugte Ausführungsbeispiele werden nachfolgend, bezugnehmend auf die beigefügten Figuren, näher erläutert.

Es zeigen:
Fig. 1 ein Flussdiagramm eines Ausführungsbeispiels eines Verfahrens zum Bewerten eines Verlaufs einer Trainingsübung;
Fig. 2 ein Beispiel für eine grafische Repräsentation eines Übungsareals sowie der an der Trainingsübung beteiligten Objekte;
Fig. 3 ein Beispiel für eine grafische Anzeige, die die Bewertung des Verlaufs der Trainingsübung repräsentiert; und
Fig. 4 eine schematische Darstellung eines Ausführungsbeispiels eines Analysators zum Bewerten eines Verlaufs einer Trainingsübung.

Das Verfahren zum automatischen Bewerten eines Verlaufs einer Trainingsübung umfasst ein Bereitstellen 10 einer Mehrzahl von Ortsinformationen für zumindest ein an der Trainingsübung teilnehmendes bewegliches Objekt sowie ein weiteres Bereitstellen 20 zumindest eines vorbestimmten Übungsziels. Während der Auswertung wird ein Vergleich 30 der Mehrzahl von Ortsinformationen mit dem Übungsziel vorgenommen bzw. es werden die Ortsinformationen in Bezug auf das Übungsziel ausgewertet. Dabei können die Ortsinformationen entweder direkt mit einem Übungsziel verglichen werden oder es können basierend auf den Ortsinformationen von diesen abgeleitete Größen bestimmt werden, die dann einem Vergleich zugrunde gelegt werden, beispielsweise eine Geschwindigkeit, eine Beschleunigung, eine mittlere Rate von Richtungsänderungen pro Zeiteinheit oder dergleichen. Weitere Beispiele für zu ermittelnde Größen sind Sprintzeiten für eine vorbestimmte Strecke oder die sogenannte Tappingfrequenz, die eine Anzahl von Schritten eines Sportlers angibt, die dieser auf einer Stelle pro Sekunde durchführen kann. Als ein Übungsziel könnte beispielsweise eine Tappingfrequenz von 15 pro Sekunde oder eine Sprintzeit von 10 Sekunden für einen Sprint von 100 Metern definiert werden. Zum Bestimmen der Tappingfrequenz kann beispielsweise eine Mehrzahl von Sensoren an einem Sportler befestigt werden, welche jeder mehrere Ortsinformationen pro Zeiteinheit liefern sodass beispielsweise auch die Orientierung von Gliedmaßen eines Sportlers bestimmt werden kann. Mit anderen Worten kann die Mehrzahl von Ortsinformationen für zumindest ein an der Trainingsübung teilnehmendes bewegliches Objekt von mehreren Sensoren herrühren. Beim Bewerten 40 des Verlaufs der Trainingsübung wird die Bewertung basierend auf dem Ergebnis des Vergleiches 30 durchgeführt. Es könnte beispielsweise als positiv bewertet werden, wenn ein einzelnes Übungsziel erreicht wurde oder wenn bei mehrfachem Wiederholen einer Übung das Übungsziel zu mehr als einem vorbestimmten Prozentsatz erreicht wurde.

Dabei ist die Reihenfolge dieser Verfahrensschritte nicht festgelegt, beispielsweise kann das vorbestimmte Übungsziel bzw. die Übungsziele, die zur Bewertung des Verlaufs der Trainingsübung zu verwenden sind, bereitgestellt werden, bevor die Ortsinformationen bereitgestellt oder erhoben werden. Ebenso können ein oder mehrere Übungsziele auch erst nach dem Durchführen einer realen Übung bereitgestellt bzw. definiert werden.

Das Bereitstellen des zumindest einen Übungsziels bzw. der typischerweise mehreren Übungsziele, anhand derer die Trainingsübung bewertet wird, kann auch als Erstellen einer Übungsschablone bezeichnet werden. Dies kann unter anderem in Verbindung mit der Definition des während der Trainingsübung verwendeten Materials erfolgen, also zusammen mit einer Definition der beweglichen und unbeweglichen Objekte, die innerhalb der Trainingsübung verwendet werden bzw. Teil derselben sind. Das Bereitstellen der vorbestimmten Übungsziele kann also die Definition der Objekte und die Definition des verwendeten Materials umfassen, sowie eine Definition weiterer Auswertelogik, also beispielsweise eine Auswahl auszuwertender Parameter, wie beispielsweise Zwischenzeiten, Laufwege, die Anzahl von Pässen oder dergleichen. Des Weiteren kann das Bereitstellen eines Übungsziels die Definition des Zweckes einer Übung umfassen, sowie eine Definition eines Areals bzw. eines vorbestimmten Übungsareals und der beteiligten Teams. Zur Definition der beteiligten Teams können beispielsweise die mittels eines automatischen Positionsverfolgungssystems gewonnenen Positionen einzelner Mitspieler von Teamsportarten unterschiedlichen Teams zugeordnet werden.

Mit anderen Worten kann aufgrund der großen Flexibilität der Ausführungsbeispiele eine beliebige Auswertelogik verwendet werden, um nahezu beliebigen Fragestellungen nachzugehen.

Dabei können gemäß einiger Ausführungsbeispiele beispielsweise taktische Parameter bestimmt und der Bewertung des Erfolgs einer Trainingsübung zugrunde gelegt werden. Ein Beispiel für einen taktischen Parameter ist die Form der von einem Teilnehmer an der Trainingsübung zurückgelegten Trajektorie. Die Bewertung des Erfolges kann beispielsweise erfolgen, indem vorgegebene Sollaufwege bzw. Trajektorien für bestimmte Spielzüge, beispielsweise beim Fußball, Football, Eishockey, Handball etc., mit den tatsächlich bestimmten Trajektorien verglichen werden. Eine weitere Vorgabe für eine Auswertelogik könnte sein, dass eine vorgegebene Zone von einem oder mehreren Teilnehmern an der Trainingsübung nicht verlassen werden sollen, oder die Aufenthaltsdauer eines Teilnehmers im Deckungsschatten eines anderen Teilnehmers bewertet werden soll, wobei eine kürzere Aufenthaltsdauer besser bewertet werden kann, als eine längere. Beispiele für weitere taktische Parameter sind die Abstände zwischen Übungsmaterial und Mitspielern bzw. zwischen Mitspielern selbst. Ein Übungsziel könnte beispielsweise sein, dass die Abstände innerhalb einer Viererkette eingehalten werden sollen, wobei eine Rückmeldung erfolgt, wenn diese zu groß oder zu klein werden. Weitere mögliche Parameter sind der Abstand zwischen georteten Stangen bzw. Hütchen, die in einem engen Slalom umlaufen werden sollen oder die Größe eines von einem Teilnehmer oder eine Mannschaft abgedeckten Raums sowie die Kompaktheit (= Fläche) einer Mannschaft, die beispielsweise durch ein die Mannschaft umgebendes Polygon gemessen werden könnte.

Gemäß weiteren Ausführungsbeispielen können Sportartspezifische technische Parameter bestimmt und der Bewertung des Erfolgs einer Trainingsübung zugrunde gelegt werden. Ein Beispiel dafür wären Pässe und deren Eigenschaften, beispielsweise die Anzahl von Pässen pro Zeiteinheit, die Geschwindigkeit eines Passes, die Zeit im Ballbesitz vor einem Pass. Wird beispielsweise als ein Übungsziel das Forcieren eines schnellen Spiels definiert, sollte die Zeit vor dem Pass im Mittel unter einem bestimmten Schwellwert liegen. Weitere zu bestimmende Parameter können beispielsweise die Anzahl der Ballberührungen vor einem Pass (direkte Pässe könnten eine Übungsziel sein, also eine Ballberührung) sowie das Verhältnis der erfolgreichen Pässe (die einen Mitspieler erreichen) zu den nicht erfolgreichen Pässen (die einen Gegenspieler erreichen oder ins "Aus" gehen) sein. Weitere Beispiele für eine Ballsportart können die Ballkontakte betreffen, z.B. die Anzahl von Ballkontakten mit rechtem bzw. linkem Fuß und deren Vergleich mit einer vom Übungsziel umfassten Vorgabe. Weitere Beispiele betreffen Torschüsse und deren Eigenschaften, wie beispielsweise die Schussgeschwindigkeit, die Zone im Tor, die getroffen werden soll bzw. wurde oder die Torentfernung für einen Abschluss. Als Verallgemeinerung dazu können bestimmte Abstände oder Zonen als vorgegebene Grenzen vorgegeben und der Bewertung zugrunde gelegt werden. Im Fall der Bewertung eine Freistoßübung könnte beispielsweise als ein Übungsziel eine gewünschte Trajektorie für eine Ball vorgeben werden, beispielsweise links/rechts an einer Mauer vorbei oder unter einer Mauer hindurch.

Gemäß weiteren Ausführungsbeispielen können athletische Parameter bestimmt werden, wie beispielsweise die von einem Teilnehmer der Trainingsübung zurückgelegte Wegstrecke. Im Rehatraining könnte eine Trainingsübung dann beispielsweise als erfolgreich bewertet werden, wenn eine vorgegeben Wegstrecke zurückgelegt wurde. Als weiterer athletischer Parameter könnte beispielsweise die Geschwindigkeit bestimmt werden. Gemäß der Auswertelogik könnte dann beispielsweise bestimmt werden, wie oft eine Maximalgeschwindigkeit erreicht wurde, wie nahe ein Teilnehmer der geforderten Geschwindigkeit gekommen ist oder ob eine vorbestimmte Geschwindigkeit nicht überschritten oder unterschritten wurde. Als weiterer athletischer Parameter könnte beispielsweise die Schrittlänge und die Schrittfrequenz bestimmt werden. Gemäß der Auswertelogik könnte dann beispielsweise bestimmt werden, ob das Ziel, kurze, schnelle Schritte zu verwenden, erreicht wurde oder nicht. Gemäß der Auswertelogik könnte ferner die Laufintensität bestimmt werden, um zu bewerten, ob das Übungsziel Sprint, schnelles Laufen, mittelschnelles Laufen, langsames Laufen, Gehen oder Stehen erreicht wurde oder ob in einer komplexeren Trainingsübung eine vorgegebene Abfolge an Laufintensitäten eingehalten wurde.

Das Definieren der Übungsschablone kann gemäß einigen Ausführungsbeispielen auch das Bereitstellen zumindest einer Ortsinformation für eine zeitlich unveränderliche Position innerhalb des Übungsareals umfassen, die zu keinem physikalischen Objekt korrespondiert. Diese Ortsinformation kann als zu einem virtuellen Material korrespondierend bezeichnet werden. Ein Beispiel dafür ist eine virtuelle Linie oder ein virtueller Punkt innerhalb des Übungsareals, sofern dieser für einen gewollten bzw. vorbestimmten Verlauf der Trainingsübung wichtig ist. Beispielsweise kann eine derartige virtuelle Linie anzeigen, wenn ein Spieler aus einem zulässigen Übungsbereich herausgelaufen ist, was beispielsweise zu einem erfolglosen Abbruch der Übung führen kann bzw. derart gewertet werden könnte. Ebenso könnte eine solche Linie als Start- und/oder End- Kriterium für z.B. Zeitmessungen dienen.

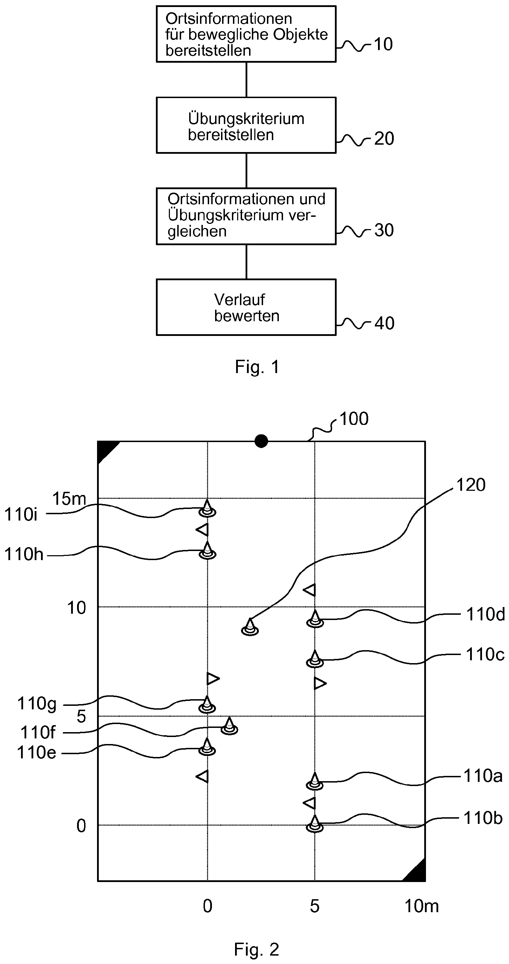

Fig. 2 zeigt eine grafische Repräsentation eines Übungsaufbaus innerhalb eines Übungsareals 100. Übungsobjekte 110a bis 110j innerhalb des Übungsareals 100 dienen als Beispiel für eine von vielen Möglichkeiten, wie Übungsobjekte bereitgestellt bzw. visualisiert werden können. Dabei sind in Fig. 2 innerhalb des umrandet dargestellten vorbestimmten Übungsareals 100 mehrere Übungsobjekte 110a-110j gezeigt. Lediglich als Beispiel für Übungsobjekte 100 sind hier Hütchen oder Pylonen genannt, in dem die Sportler während einer Trainingsübung laufen sollen und die zu realen Objekten korrespondieren. Ebenso zeigt Fig. 2 die Auswertelogik, wie z.B. eine angedeutete Lichtschranke zwischen Objekt 110h und 100i, bzw. zwischen Objekt 110a und 110b mit einem Auslöser in Pfeilrichtung. Des Weiteren sind an 110c, 110d, 110e und 110g Lichtschranken an die Hütchen einseitig gebunden, d.h. sie bewegen sich mit den Positionen der realen Hütchen, verändern aber ihre relative Lage dazu nicht. Lichtschranke (110a -> 110b) wird dabei als Startlichtschranke, Lichtschranke (110h -> 110i) als Endlichtschranke definiert. Alle weiteren Lichtschranken dienen als Zwischenzeitgeber, die in beliebiger Reihenfolge definiert werden könnten.

Die Übungsschablone kann beispielsweise zunächst lediglich virtuell vorbereitet, d.h., mittels einer grafischen Benutzerschnittstelle angelegt werden. Alternativ oder ergänzend können die Materialen bzw. die bei der Übung verwendeten Übungsobjekte gleichzeitig auch auf einem realen Übungsareal platziert bzw. aufgestellt werden, welches zu der grafischen Darstellung der Fig. 2 korrespondiert. Dazu können Übungsobjekte an den virtuell vorbestimmten Positionen aufgestellt werden oder es können Übungsobjekte auf dem realen Übungsareal aufgestellt werden, deren Position dann gemäß nicht zur Erfindung gehörenden Beispielen mittels eines Positionserfassungssystems in die grafische Repräsentation der Fig. 2 übernommen wird. Dies kann auch als automatisches "Snapping" von getracktem realen Material bzw. von Übungsobjekten bezeichnet werden.

Eine ebenfalls nicht zur Erfindung gehörende Alternative ist das Vorbereiten der Übung bzw. das Erstellen der Schablone einer Übung, ohne dass reales Material innerhalb eines Platzes bzw. des Übungsareals 100 vorhanden ist. Das ungetrackte Material bzw. die unbe weglichen Objekte können anschließend sowohl in der grafischen Benutzerschnittstelle als auch real noch verschoben bzw. fein justiert werden. Dies kann beispielsweise dadurch erfolgen, dass eine vorbereitete Schablone einer Trainingsübung so lange verschoben wird, bis diese mit den von den realen Übungsobjekten bestimmten Objekten übereinstimmt, wodurch die Schablone auf den realen Positionen einrastet. Zur Vereinfachung kann die Schablone dabei skaliert werden oder es kann eine automatische oder manuelle Translation einzelner Objekte vorgenommen werden, um Abweichungen der Schablone von dem realen Aufbau teilweise automatisch auszugleichen. Zum selben Zweck können Teile oder die gesamte Schablone möglicherweise auch rotiert werden.

Insofern mehrere bewegliche Objekte bzw. Sportler an der Übung teilnehmen, können mehrere Sportler unterschiedlichen Teams zugeordnet werden. Dies kann entweder manuell geschehen, in denen einzelne bewegliche Objekte angeklickt bzw. markiert und zu Teams zusammengestellt werden oder indem mehrere bewegliche Objekte gleichzeitig markiert und so als zu einem Team gehörig gekennzeichnet werden. Eine mögliche Alternative ist die automatische Zuordnung zu Teams, beispielsweise anhand der initialen Aufstellung der Sportler bzw. der beweglichen Objekte, anhand des Durchschreitens bestimmter Tore, seien sie real oder virtuell, oder das Aufstellen der beweglichen Objekte bzw. der einzelnen Sportler an bestimmten Markern oder in bestimmten Feldern.

Während die vorherigen Überlegungen das Verfahren zum automatischen Bewerten eines Verlaufes einer Trainingsübung betrafen, wird in den nachfolgenden Absätzen kurz zusammengefasst, wie die Voraussetzungen für die real abzuhaltende Trainingsübung auf einem Trainingsplatz bzw. innerhalb eines vorbestimmten Übungsareals geschaffen werden können. Zunächst können die unbeweglichen Objekte, die vorher definiert wurden, innerhalb des Übungsareals angeordnet werden. Dazu können beispielsweise akustische oder visuelle Rückmeldungen für das korrekte Platzieren von getracktem Trainingsmaterial, also von solchen Objekten, deren Positionen auch mittels eines automatischen Positionserfassungssystems bestimmt werden können, erfolgen. Dies kann beispielsweise durch ein akustisches Feedback unterstützt werden, bei dem die Frequenz eines Piepsens schneller wird, je näher sich der zu platzierende unbewegliche Gegenstand an seiner gewünschten Position befindet. Die gewünschte Position kann beispielsweise mithilfe eines Dauertons angezeigt werden. Eine andere Möglichkeit wäre eine Ansage durch eine computergestützte Sprachsynthese. Alternativ kann der Aufbau der realen Übung durch andere Maßnahmen unterstützt werden, beispielsweise durch das Projizieren der gewünschten Positionen innerhalb des realen

Übungsareals, beispielsweise mittels eines Beamers, eines Videobodens oder durch Lasermarkierungen.

Das Bereitstellen der Ortsinformationen für die getrackten beweglichen Objekte kann durch Aufzeichnen der Positionen zu aufeinander folgenden Zeitpunkten erfolgen. Dazu kann die Übung beispielsweise manuell gestartet werden, d.h., der Beginn der Aufzeichnung kann manuell ausgelöst werden. Alternativ kann die Übung auch automatisiert gestartet werden, indem beispielsweise ein vorkonfiguriertes Ereignis von zumindest einem beweglichen Objekt beobachtet wird. Dies kann beispielsweise das Überqueren einer virtuellen Linie eines beweglichen Objektes bzw. eines Spieler innerhalb des Übungsareals sein. Als Beispiel für eine solche virtuelle Linie kann die Verbindungslinie zwischen den Objekten 110a und 110b in Fig. 2 betrachtet werden. Das durchqueren dieser virtuellen Lichtschranke kann beispielsweise eine Aufzeichnung von Ortsinformationen starten oder den Beginn einer Trainingsübung angeben.

Nach Beenden der realen Übung ist die Bestimmung oder Aufzeichnung der Mehrzahl von Ortsinformationen für die an der Trainingsübung teilnehmenden beweglichen Objekte bzw. Sportler oder Spieler abgeschlossen, sodass diese Informationen dem Verfahren zum automatischen Bewerten der Trainingsübung zugeführt bzw. an dieses übergeben werden können. Alternativ kann die Bewertung ohne Aufzeichnung erfolgen, wenn die Ortsinformationen in Echtzeit an das Verfahren zum automatischen Bewerten übergeben werden. Es werden dann lediglich die Bewertungsergebnisse für spätere Anzeigen gespeichert.

Durch den Vergleich der Positionsinformationen mit den Übungszielen wird daraufhin der Verlauf der Trainingsübung bestimmt. Dabei können ohne Anspruch auf Vollständigkeit beispielsweise Durchläufe von Sportlern bzw. die Anzahl von Runden, die ein Sportler innerhalb eines Übungsparcours absolviert hat, bestimmt werden. Des Weiteren können Zeiten, Distanzen und dergleichen bestimmt werden. Auch können bestimmte Ereignisse bzw. deren Eintreten ein Indiz für die Bewertung einer Trainingsübung sein, beispielsweise das Erreichen bestimmter Durchlauf-/Zwischenzeiten oder das Einhalten von vorbestimmten Rundenzeiten bzw. pro Zeiteinheit zurückzulegenden Distanzen sowie das Einhalten von vorbestimmten Abständen zu Übungsmaterialien oder dergleichen.

Nach Bewerten des Verlaufs der Trainingsübung kann entweder die Bewertung selbst zur weiteren Verwendung und Analyse in der Datenbank gespeichert werden oder es können die Übungsziele sowie die aufgezeichneten bzw. bereitgestellten Ortsinformationen in einer Datenbank oder in einem Speichersystem abgelegt werden, um zu einem späteren Zeitpunkt erneut eine Bewertung des Verlaufs der Trainingsübung vornehmen zu können.

Ebenso wie der Beginn einer realen Übung kann auch das Ende einer realen Trainingsübung manuell bzw. automatisiert erfolgen, beispielsweise wenn ein vorbestimmtes oder vorkonfiguriertes Ereignis eintritt. Dies kann beispielsweise das Verlassen des Übungsareals 100 durch ein bewegliches Objekt bzw. durch einen Spieler sein oder das Überqueren einer weiteren virtuellen Lichtschranke, beispielsweise zwischen den Objekten 110h und 110i.

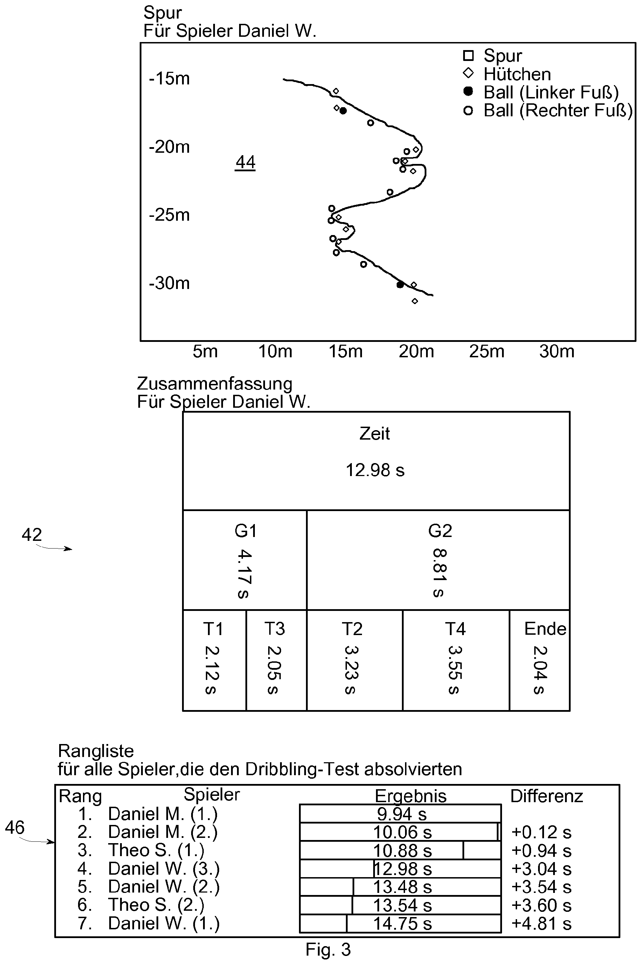

Gemäß einigen Ausführungsbeispielen wird das Ergebnis der Bewertung auch grafisch dargestellt bzw. visualisiert. Fig. 3 zeigt schematisch eine Möglichkeiten der Visualisierung der Auswertung bzw. der Bewertung der Trainingsübung.

Als Beispiel für eine mögliche Visualisierung zeigt Fig. 3 in der mittleren Darstellung 42 mehrere erzielte Zwischenzeiten und die erreichte Endzeit eines in der mittleren Darstellung visualisierten Dribblings eines Fußballspielers in tabellarischer Form. In der oberen Darstellung 44 ist eine Trajektorie 115 des Fußballs 20 als bewegliches Objektes 120 dargestellt, d.h. der zeitliche Verlauf der Position des beweglichen Objektes 120 relativ zu der Position einer Mehrzahl von weiteren Übungsobjekten 110a - 110i. In dem in Fig. 3 illustrierten Beispiel werden die Mehrzahl von Ortsinformationen für einen Fußball 120 als bewegliches Objekt verfolgt bzw. bereitgestellt. Als weiteres bewegliches Objekt könnte in weiteren Ausführungsbeispielen zusätzlich beispielsweise die Position eines mit dem Ball dribbelnden Spielers verfolgt werden. In der unteren Darstellung 46 werden die Endzeiten einer Mehrzahl von Teilnehmern an der Trainingsübung in tabellarischer Darstellung relativ zueinander verglichen, um visuell eine Rückmeldung zu geben, welcher der Teilnehmer die Trainingsübung mit der besten Leistung beendet hat.

Weitere, aus Gründen der Übersichtlichkeit nicht dargestellte Formen der Visualisierung sind beispielsweise die Darstellung in tabellarischer Form, die Darstellung von Grafen, Heatmaps, Traces oder Ähnlichem. Des Weiteren können gemäß weiteren Ausführungsbeispielen grafisch beispielsweise Querschnittvergleiche zwischen verschiedenen Sportlern oder Längsschnittvergleiche zwischen mehreren Durchläufen einer Übung desselben Sportlers dargestellt werden. Des Weiteren können beispielsweise Längsschnittvergleiche zwischen mehreren Durchläufen eines Sportlers über mehrere Durchführungen der gleichen Übungsschablone dargestellt werden.

Gemäß einigen Ausführungsbeispielen kann ein Export der Daten durchgeführt werden, wie bereits oben beschrieben. Dies kann es ermöglichen, die Trainingsübung mit weiteren, zuvor nicht konfigurierten Auswertelogiken bzw. Übungszielen zu ergänzen.

Ein Abspeichern der Ergebnisse bzw. der Bewertung in einer Datenbank kann entweder bereits zur Laufzeit oder nach Abschluss der Analyse bzw. der Bewertung des Erfolges geschehen. Wie bereits in den vorhergehenden Absätzen angesprochen, ist die Reihenfolge bei der Durchführung der Schritte des Verfahrens zum automatischen Bewerten eines Erfolges eines Verlaufs einer Trainingsübung nicht vorgegeben. Beispielsweise können gemäß einigen Ausführungsbeispielen auch zunächst die Ortsinformationen bereitgestellt bzw. die Positionen aufgezeichnet werden, bevor eine Übungsschablone erstellt wird oder die Übungsziele definiert werden. Eine Übung kann zunächst gewissermaßen ohne definiertes Ziel durchgeführt und aufgezeichnet werden, woraufhin im Nachhinein die Definition erfolgt, wie die Bewertung des Erfolges der Durchführung der Übung vorgenommen werden soll.

Die einzelnen Komponenten bzw. Teilaspekte eines Ausführungsbeispiels eines Verfahrens zum automatischen Bewerten eines Erfolges eines Verlaufs einer Trainingsübung werden nachfolgend erneut und in anderen Worten zusammengefasst.

In einer Editor-Einheit kann ein Benutzer Übungsschablonen erstellen. Für die Übungsschablone werden das Areal einer Übung und das benutzte Material festgelegt. Material kann gegebenenfalls vorplatziert werden. Alle Platzierungen sind dabei relativ zum Übungsareal. Das Übungsareal kann an jede einer Tracking-Einheit bzw. einem Positionsverfolgungssystem zugängliche absolute Position verschoben werden. Des Weiteren legt der Benutzer die Auswertelogik fest, d.h. er bestimmt, was in der Übung analysiert werden soll. Dies kann z.B. das Festlegen von Lichtschranken und deren Durchlaufreihenfolge sein. Lichtschranken können auch als "virtuelle Lichtschranken" ausgeführt sein. Eine "virtuelle-Lichtschranke" besteht dabei aus einer Verbindung zweier Koordinaten, die bei Übertreten (eines Tags bzw. einer Person mit einem Tag) in einer bestimmte Richtung oder in beiden Richtungen ein Ereignis im Analyzer auslösen kann. Die Koordinaten können dabei auch an Material bzw. unbewegliche Objekte gebunden werden, um z.B. Lichtschranken zwischen zwei Hütchen oder Lichtschranken zwischen einem Hütchen und einer dazu relativen oder absoluten Koordinate an die reale Position eines getrackten Objekts zu binden. Weiterhin können Analysen, wie z.B. die Auswertung von Ballkontakten, Sportlerwechsel eines bestimmten Materials durchgeführt werden (z.B. Pass zwischen zwei Spielern im Fußball, Übergabe des Stabes beim Staffellauf).

Die so bestimmte Übungsschablone wird auf einem Übungsareal platziert. Die Tracking-Einheit bzw. ein Positionsbestimmungssystem kann zu diesem Zeitpunkt bereits Tracking-Daten liefern, mit deren Hilfe die Übung automatisch bzw. semi-automatisch (durch Platzieren des Benutzers und automatischem "Einrasten" aufgepacktes Material/Linien/etc.) platziert bzw. aufgebaut werden kann. Dabei werden den im Editor festgelegten Objekten reale Instanzen getrackter Objekte zugewiesen.

Eine platzierte Übung wird dann entweder durch Benutzerinteraktion oder automatisch durch ein im Editor festgelegtes Ereignis gestartet. Das heißt, die Übung wird aktiv geschaltet. Automatisch kann dies z.B. durch das Übertreten einer als Start definierten Lichtschranke ausgeführt werden. Eine Übung kann auch ohne Schablone gestartet werden. Werden dabei die Tracking-Daten aufgezeichnet, können im Nachhinein Übungsschablonen erstellt, platziert und ausgewertet werden.

Während eine Übung aktiv ist, können bereits relevante Ereignisse ausgewertet, gespeichert und dargestellt werden. Dies können z.B. Rundenzeiten, Ballkontakte, Übertretungen von Lichtschranken oder Linien, Erkennen von Pässen oder Ähnliches sein. Dem Benutzer können diese Ergebnisse visualisiert werden.

Wie der Start kann das Ende einer Übung durch manuelles Stoppen durch den Benutzer, oder durch ein in der Übungsschablone festgelegtes Ereignis signalisiert werden.

Während der Übung oder nach Abschluss einer Übung können die Ereignisse innerhalb der Übung visualisiert werden. Dies kann durch Tabellen, Graphen, Heatmaps, Traces oder anderen Visualisierungen geschehen. Der Benutzer kann durch Interaktivität einzelne Sportler und Ergebnisse innerhalb einer Übung auswählen und einander gegenüber stellen (Querschnitt-Analyse). Damit sind z.B. Rankings von Durchläufen oder Sortierung nach bestimmten Ergebnissen (wie z.B. Maximale/Minimale Distanz) möglich. Ebenso ist es möglich den Längsschnitt bestimmter Sportler zu visualisieren, d.h. die Ergebnisse eines Sportlers für eine Übungsschablone von mehreren Durchführungen (innerhalb einer Übung oder in der Vergangenheit durchgeführten Übungen) auszuwerten und zu visualisieren. Dem Benutzer ist es zudem möglich, weitere neue Analysen hinzuzufügen. Die Analyzer- Einheit muss gegebenenfalls die Übung neu analysieren. Der Benutzer kann durch Auswählen eines Ereignisses bzw. Ereigniszeitraums in der (gefilterten) Liste von Ereignissen innerhalb einer Übung zum entsprechenden Zeitpunkt in einem Replay bzw. einer wiederholten Wiedergabe der Übung mittels einer graphischen Repräsentation der Übung gelangen, wie Sie beispielsweise in Figur 2 gezeigt ist. Möglich ist auch die Verknüpfung des Replays/Ereignissen mit realen Videodaten, als mit der Wiedergabe einer Video- Aufzeichnung der Durchführung der Übung.

Alle Ergebnisse einer Übung können bereits während der Durchführung der Übung oder nach dem Deaktivieren der Übung abgespeichert werden. Die Daten können z.B. für Längsschnitt-Visualisierungen benutzt werden.

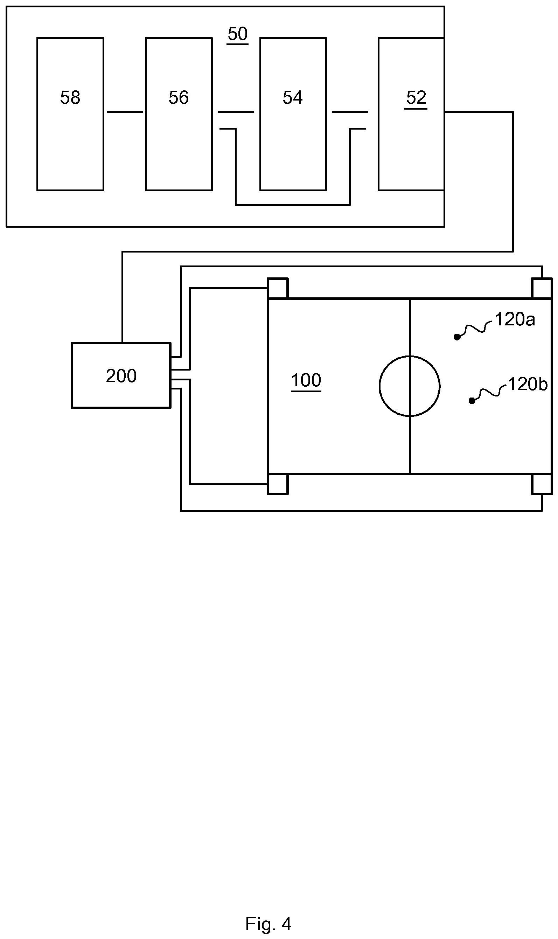

Fig. 4 zeigt erneut schematisch ein Ausführungsbeispiel eines Analysators 50 zum Bewerten des Verlaufs einer Trainingsübung. Der Analysator 50 umfasst eine Eingangsschnittstelle 52. Diese ist ausgebildet, um eine Mehrzahl von Ortsinformationen für zumindest ein an der Trainingsübung teilnehmendes bewegliches Objekt bereitzustellen oder alternativ von extern zu empfangen. Ein Speicher 54 ist ausgebildet, um zumindest ein vorbestimmtes Übungsziel bereitzustellen. Ein Vergleicher 56, der mit der Eingangsschnittstelle 52 und dem Speicher 54 verbunden ist, ist ausgebildet, um die Mehrzahl von Ortsinformationen mit dem Übungsziel zu vergleichen und das Ergebnis des Vergleichs einem Bewerter 58 zu übermitteln. Der Bewerter 58 wiederum ist ausgebildet, um den Verlauf der Trainingsübung basierend auf dem Ergebnis des Vergleichs zu bewerten und die Bewertung einen Benutzer des Systems bzw. des Analysators gemäß einem der oben beschriebenen Möglichkeiten zur Verfügung zu stellen.

Wie in Fig. 4 schematisch dargestellt, kann der Analysator 50 optional Teil eines Systems zum Bewerten eines Verlaufs einer Trainingsübung sein, welches ferner ein Positionserfassungssystem 200 umfasst, das ausgebildet ist, um Ortsinformationen für zumindest ein an der Trainingsübung teilnehmendes bewegliches Objekt innerhalb eines Übungsareals 100 zu erfassen. Zu diesem Zweck kann das Positionserfassungssystem 200 beispielsweise über eine Mehrzahl von Funkempfängern oder -sendern 220a- 220d verfügen, mittels derer die Position beweglicher Objekte 120a oder 120b innerhalb des Übungsareals 100 bestimmt bzw. getrackt werden können. Lediglich als Beispiel ist in Fig. 4 ein Fußballfeld als Übungsareal 100 angedeutet, auf dem die Position zweier Spieler 120a und 120b vom Positionserfassungssystem 200 bestimmt wird. Bei weiteren Ausführungsbeispielen können auch lediglich Teile des Feldes als Übungsgelände verwendet werden. Das Positionserfassungssystem 200 kann ebenfalls die Ortsinformationen über die Zeit zumindest einem an der Trainingsübung teilnehmendem Objekt innerhalb eines Übungsareals 100 speichern und gegebenenfalls zu einem späteren Zeitpunkt wieder abspielen.

Ausführungsbeispiele sind ein Verfahren zum automatischen Bewerten eines Verlaufes einer Trainingsübung, umfassend ein Bereitstellen einer Mehrzahl von Ortsinformationen für zumindest ein an der Trainingsübung teilnehmendes bewegliches Objekt; ein Bereitstellen zumindest eines vorbestimmten Übungsziels; ein Bereitstellen zumindest einer weiteren Ortsinformationen unter Verwendung einer graphischen Benutzerschnittstelle, die eine graphischen Repräsentation eines Übungsareals sowie eine graphische Repräsentation eines zu der zumindest einen weiteren Ortsinformation korrespondierenden Übungsobjektes in einer grafischen Anzeige umfasst, wobei die weitere Ortsinformation die Position eines an der Trainingsübung beteiligten Übungsobjektes innerhalb des Übungsareals oder eine zeitlich unveränderliche Position innerhalb des Übungsareals angibt, welche zu einem vorbestimmten Verlauf der Trainingsübung korrespondiert; ein Vergleichen der Mehrzahl von Ortsinformationen mit dem Übungsziel; und ein Bewerten des Verlaufs der Trainingsübung basierend auf dem Ergebnis des Vergleiches, wobei das Bereitstellen des zumindest einen vorbestimmten Übungsziels ein Auswählen zumindest einer verwendeten Auswertelogik für die Ortsinformationen aus einer Mehrzahl zur Verfügung stehender Auswertelogiken umfasst.

Gemäß einigen dieser Ausführungsbeispiele zeigen die Ortsinformationen für ein Objekt jeweils eine Position des Objektes innerhalb eines vorbestimmten Übungsareals an.

Gemäß einigen dieser Ausführungsbeispiele werden die Position durch die Koordinaten des Objektes in einem beliebigen der Trainingsübung zugeordneten Koordinatensystem angegeben ist.

Gemäß einigen dieser Ausführungsbeispiele umfasst das Bereitstellen der weiteren Ortsinformationen eine Benutzereingabe der Position.

Gemäß einigen dieser Ausführungsbeispiele wird die graphische Repräsentation des Übungsobjektes auf der grafischen Anzeige ausgehend von einer vordefinierten Initialposition derart verändert wird, dass die Position der graphische Repräsentation des Übungsobjektes innerhalb der graphischen Repräsentation des Übungsareals der mittels des Positionsverfolgungssystems bestimmten Position des Übungsobjektes innerhalb des Übungsareals entspricht, wobei wobei das Verändern ein verschieben der graphischen Repräsentation des Übungsobjektes relativ zu der graphischen Repräsentation des Übungsareals, eine Rotation der grafischen Anzeige oder eine Skalierung der grafischen Anzeige umfasst.

Gemäß einigen dieser Ausführungsbeispiele umfasst das Übungsziel zumindest ein Kriterium der Gruppe von Zielen, die das Eintreten des beweglichen Objektes in einen vorbestimmten Bereich innerhalb des Übungsareals, eine Interaktion des beweglichen Objektes mit einem weiteren Objekt, das Erreichen einer vorbestimmten Geschwindigkeit durch das bewegliche Objekt und die Übereinstimmung der Ortsinformationen mit einer vorbestimmten Sequenz von Ortsinformationen umfasst.

Ausführungsbeispiele sind ferner ein System zum Bewerten eines Verlaufes einer Trainingsübung, umfassend ein Positionserfassungssystem, das ausgebildet ist, um Ortsinformationen für zumindest ein an der Trainingsübung teilnehmendes bewegliches Objekt innerhalb eines Übungsareals zu erfassen; und eine Analysator, dessen Eingangsschnittstelle zum Empfang der Ortsinformationen mit dem Positionserfassungssystem verbunden ist, wobei der Analysator zum Bewerten des Verlaufes einer Trainingsübung folgendes umfasst: eine Eingangsschnittstelle, die ausgebildet ist, um eine Mehrzahl von Ortsinformationen für zumindest ein an der Trainingsübung teilnehmendes bewegliches Objekt bereitzustellen; einen Speicher, der ausgebildet ist, um zumindest eines vorbestimmten Übungsziel bereitzustellen; eine graphische Benutzerschnittstelle, die eine graphischen Repräsentation eines Übungsareals sowie eine graphische Repräsentation eines zu der zumindest einen weiteren Ortsinformation korrespondierenden Übungsobjektes in einer grafischen Anzeige umfasst, und mittels derer die weitere Ortsinformation bereitgestellt werden kann, wobei die weitere Ortsinformation die Position eines an der Trainingsübung beteiligten Übungsobjektes innerhalb des Übungsareals oder eine zeitlich unveränderliche Position innerhalb des Übungsareals angibt, welche zu einem vorbestimmten Verlauf der Trainingsübung korrespondiert; einen Vergleicher, der ausgebildet ist, um die Mehrzahl von Ortsinformationen mit zumindest einem der Trainingsübung zugeordneten vorbestimmten Übungsziel zu vergleichen; und einen Bewerter, der ausgebildet ist, um den Verlauf der Trainingsübung basierend auf dem Ergebnis des Vergleiches zu bewerten.

Wenngleich in der vorhergehenden Beschreibung überwiegend bei Anwendungen beschrieben, bei denen eine Mehrzahl von Sportlern interagiert, beispielsweise bei Fußballübungen bzw. Trainingsübungen für Fußballspieler, können weitere Ausführungsbeispiele der vorliegenden Erfindung auch in anderen Anwendungsgebieten im Sport verwendet werden. Beispielsweise können weitere Ausführungsbeispiele zur Leistungsdiagnostik beliebiger Hochleistungssportarten verwendet werden, bei denen auch lediglich ein Sportler beteiligt sein kann, wie beispielsweise bei sämtlichen leichtathletischen Disziplinen. Dazu kann zur Bewertung einer Trainingsübung beispielsweise nicht lediglich eine absolute Position eines Sportlers bzw. eines beweglichen Objektes im Raum bestimmt werden, sondern mehrere Positionen können für oder an einem einzigen Sportler bestimmt oder verwendet werden, um den Erfolg einer Trainingsübung zu bestimmen. Beispielsweise können die Extremitäten von Sportlern mit mehreren Tags bzw. Positionserfassungssensoren ausgestattet werden, sodass zusätzlich zu einer Position eines Schwerpunkts eines Sportlers auch weitere Informationen bezüglich dessen Körperhaltung berücksichtigt werden können, um den Erfolg einer Trainingsübung zu bewerten. Dies kann beispielsweise dazu verwendet werden, die Technik von Hochspringern Stabhochspringern oder von Sportlern anderer Leichtathletikdisziplinen zu beobachten und in Verbindung mit geeigneten Übungszielen zu bestimmen, ob dieser die für seine Sportart typischen Bewegungsabläufe mit hinreichender Präzision während der Trainingsübung ausgeführt hat oder nicht.

Die in der vorstehenden Beschreibung, den nachfolgenden Ansprüchen und den beigefügten Figuren offenbarten Merkmale können sowohl einzeln wie auch in beliebiger Kombination für die Verwirklichung eines Ausführungsbeispiels in ihren verschiedenen Ausgestaltungen von Bedeutung sein und implementiert werden.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar.

Je nach bestimmten Implementierungsanforderungen können Ausführungsbeispiele der Erfindung in Hardware oder in Software implementiert sein. Die Implementierung kann unter Verwendung eines digitalen Speichermediums, beispielsweise einer Floppy-Disk, einer DVD, einer Blu-Ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einer programmierbaren Hardwarekomponente derart zusammenwirken können oder zusammenwirken, dass das jeweilige Verfahren durchgeführt wird.

Eine programmierbare Hardwarekomponente kann durch einen Prozessor, einen Computerprozessor (CPU = Central Processing Unit), einen Grafikprozessor (GPU = Graphics Processing Unit), einen Computer, ein Computersystem, einen anwendungsspezifischen integrierten Schaltkreis (ASIC = Application-Specific Integrated Circuit), einen integrierten Schaltkreis (IC = Integrated Circuit), ein Ein-Chip-System (SOC = System on Chip), ein programmierbares Logikelement oder ein feldprogrammierbares Gatterarray mit einem Mikroprozessor (FPGA = Field Programmable Gate Array) gebildet sein.

Das digitale Speichermedium kann daher maschinen- oder computerlesbar sein. Manche Ausführungsbeispiele umfassen also einen Datenträger, der elektronisch lesbare Steuersignale aufweist, die in der Lage sind, mit einem programmierbaren Computersystem oder einer programmierbare Hardwarekomponente derart zusammenzuwirken, dass eines der hierin beschriebenen Verfahren durchgeführt wird. Ein Ausführungsbeispiel ist somit ein Datenträger (oder ein digitales Speichermedium oder ein computerlesbares Medium), auf dem das Programm zum Durchführen eines der hierin beschriebenen Verfahren aufgezeichnet ist.

Allgemein können Ausführungsbeispiele der vorliegenden Erfindung als Programm, Firmware, Computerprogramm oder Computerprogrammprodukt mit einem Programmcode oder als Daten implementiert sein, wobei der Programmcode oder die Daten dahin gehend wirksam ist bzw. sind, eines der Verfahren durchzuführen, wenn das Programm auf einem Prozessor oder einer programmierbaren Hardwarekomponente abläuft. Der Programmcode oder die Daten kann bzw. können beispielsweise auch auf einem maschinenlesbaren Träger oder Datenträger gespeichert sein. Der Programmcode oder die Daten können unter anderem als Quellcode, Maschinencode oder Bytecode sowie als anderer Zwischencode vorliegen.

Ein weiteres Ausführungsbeispiel ist ferner ein Datenstrom, eine Signalfolge oder eine Sequenz von Signalen, der bzw. die das Programm zum Durchführen eines der hierin beschriebenen Verfahren darstellt bzw. darstellen. Der Datenstrom, die Signalfolge oder die Sequenz von Signalen kann bzw. können beispielsweise dahin gehend konfiguriert sein, um über eine Datenkommunikationsverbindung, beispielsweise über das Internet oder ein anderes Netzwerk, transferiert zu werden. Ausführungsbeispiele sind so auch Daten repräsentierende Signalfolgen, die für eine Übersendung über ein Netzwerk oder eine Datenkommunikationsverbindung geeignet sind, wobei die Daten das Programm darstellen.

Ein Programm gemäß einem Ausführungsbeispiel kann eines der Verfahren während seiner Durchführung beispielsweise dadurch umsetzen, dass dieses Speicherstellen ausliest oder in diese ein Datum oder mehrere Daten hinein schreibt, wodurch gegebenenfalls Schaltvorgänge oder andere Vorgänge in Transistorstrukturen, in Verstärkerstrukturen oder in anderen elektrischen, optischen, magnetischen oder nach einem anderen Funktionsprinzip arbeitenden Bauteile hervorgerufen werden. Entsprechend können durch ein Auslesen einer Speicherstelle Daten, Werte, Sensorwerte oder andere Informationen von einem Programm erfasst, bestimmt oder gemessen werden. Ein Programm kann daher durch ein Auslesen von einer oder mehreren Speicherstellen Größen, Werte, Messgrößen und andere Informationen erfassen, bestimmen oder messen, sowie durch ein Schreiben in eine oder mehrere Speicherstellen eine Aktion bewirken, veranlassen oder durchführen sowie andere Geräte, Maschinen und Komponenten ansteuern.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

## Patentansprüche

1. Verfahren zum automatischen Bewerten eines Verlaufes einer Trainingsübung, umfassend:
Bereitstellen (10) einer Mehrzahl von Ortsinformationen für zumindest ein an der Trainingsübung teilnehmendes bewegliches Objekt (120; 120a, 120b), wobei das Bereitstellen der Mehrzahl von Ortsinformationen das Bestimmen der Position des beweglichen Objektes (120; 120a, 120b) innerhalb eines Übungsareals (100) mittels eines Positionsverfolgungssystems (200) zu mehreren aufeinanderfolgenden Zeitpunkten umfasst;
Bereitstellen zumindest einer weiteren Ortsinformationen unter Verwendung einer grafischen Benutzerschnittstelle, die eine grafische Repräsentation des Übungsareals (100) sowie eine grafische Repräsentation eines an der Trainingsübung beteiligten Übungsobjektes (110a -110j) in einer grafischen Anzeige umfasst, wobei die weitere Ortsinformation die Position des an der Trainingsübung beteiligten Übungsobjektes (110a - 110j) innerhalb des Übungsareals (100) angibt, und wobei das Übungsobjekt (110a - 110j) prinzipiell beweglich ist, eine Bewegung des Übungsobjekts (110a - 110j) aber nicht Ziel der Trainingsübung ist;
Bereitstellen (20) zumindest eines vorbestimmten Übungsziels, wobei das Bereitstellen (20) des Übungsziels das Definieren einer gewünschten Interaktion zwischen dem beweglichen Objekt (120) und dem an der Trainingsübung beteiligten Übungsobjekt (110a - 110j) umfasst;
Vergleichen (30) der Mehrzahl von Ortsinformationen mit dem Übungsziel; und
Bewerten (40) des Verlaufs der Trainingsübung basierend auf dem Ergebnis des Vergleiches (30), wobei das Bereitstellen (20) des zumindest einen vorbestimmten Übungsziels ein Auswählen zumindest einer verwendeten Auswertelogik für die Ortsinformationen aus einer Mehrzahl zur Verfügung stehender Auswertelogiken umfasst,
wobei das Bereitstellen der weiteren Ortsinformation ein Vergleichen der Position der grafischen Repräsentation des Übungsobjektes (110a -110j) innerhalb der grafischen Repräsentation des Übungsareals (100) mit einer mittels des Positionsverfolgungssystems bestimmten Position des Übungsobjektes innerhalb des Übungsareals umfasst, und
wobei das Verfahren ferner ein Bereitstellen eines akustischen, visuellen oder haptischen Signals durch das Positionsverfolgungssystem, das eine Information über eine Abweichung zwischen der mittels des Positionsverfolgungssystems bestimmten Position des Übungsobjektes innerhalb des Übungsareals und der Position der grafischen Repräsentation des Übungsobjektes (110a -110j) innerhalb der grafischen Repräsentation des Übungsareals (100) umfasst, so dass die Position des Übungsobjektes innerhalb des Übungsareals beim Aufbau der Trainingsübung derart verändert werden kann, dass die mittels des Positionsverfolgungssystems bestimmte Position des Übungsobjektes innerhalb des Übungsareals der Position der grafischen Repräsentation des Übungsobjektes (110a -110j) innerhalb der grafischen Repräsentation des Übungsareals (100) entspricht, umfasst.

2. Verfahren nach Anspruch 1, wobei gemäß der verwendeten Auswertelogik basierend auf den Ortsinformationen ein taktischer Parameter oder ein technischer Parameter für eine Sportart bestimmt wird.

3. Verfahren nach Anspruch 1, wobei gemäß der verwendeten Auswertelogik basierend auf den Ortsinformationen ein athletischer Parameter für zumindest eine an der Trainingsübung beteiligte Person bestimmt wird.

4. Verfahren nach einem der vorhergehenden Ansprüche, wobei das Bereitstellen der weiteren Ortsinformation ein Bewegen der grafischen Repräsentation des Übungsobjektes (110a -110j) auf der grafischen Anzeige umfasst.

5. Verfahren nach einem der vorhergehenden Ansprüche, wobei das Bereitstellen (20) des vorbestimmten Übungsziels eine Definition von Parametern umfasst, die für das bewegliche Objekt (120) basierend auf den Ortsinformationen bestimmt werden, um den Verlauf der Trainingsübung zu bewerten.

6. Verfahren nach einem der vorhergehenden Ansprüche, bei dem das Bereitstellen (20) des Übungsziels eine Definition aller beweglichen Objekte (120) und aller Übungsobjekte (110a-110j) umfasst, die an der Trainingsübung beteiligt sind.

7. Verfahren nach einem der vorhergehenden Ansprüche, wobei das Bewerten des Verlaufs der Trainingsübung ein Ausgeben einer visuellen Darstellung der Ergebnisse des Vergleiches auf einer Anzeige umfasst.

8. System zum Bewerten eines Verlaufes einer Trainingsübung, umfassend:
ein Positionserfassungssystem (200), das ausgebildet ist, eine Position eines beweglichen Objektes (120; 120a, 120b) innerhalb eines Übungsareals (100) zu mehreren aufeinanderfolgenden Zeitpunkten zu bestimmen, um eine Mehrzahl an Ortsinformationen für das bewegliche Objekt (120a, 120b) bereitzustellen, und eine Position eines Übungsobjektes innerhalb des Übungsareals (100) zu bestimmen, wobei das Übungsobjekt (110a - 110j) prinzipiell beweglich ist, eine Bewegung des Übungsobjekts (110a - 110j) aber nicht Ziel der Trainingsübung ist; und
einem Analysator (50) mit einer Eingangsschnittstelle (52), die mit dem Positionserfassungssystem (200) zum Empfang der Mehrzahl an Ortsinformationen und der durch das Positionserfassungssystem (200) bestimmten Position des Übungsobjektes verbunden ist,
wobei der Analysator ferner Folgendes umfasst:
eine grafische Benutzerschnittstelle, die eine grafischen Repräsentation des Übungsareals (100) sowie eine grafische Repräsentation des Übungsobjektes (110a -110j) in einer grafischen Anzeige umfasst, und mittels derer eine weitere Ortsinformation bereitgestellt wird, wobei die weitere Ortsinformation die Position des an der Trainingsübung beteiligten Übungsobjektes (110a - 110i) innerhalb des Übungsareals (100) angibt;
einen Speicher (54), der ausgebildet ist, um zumindest ein vorbestimmtes Übungsziel bereitzustellen, wobei das Übungsziel eine gewünschte Interaktion zwischen dem beweglichen Objekt (120) und dem an der Trainingsübung beteiligten Übungsobjekt (110a - 110j) definiert, wobei das zumindest eine vorbestimmte Übungsziel zumindest eine aus einer Mehrzahl zur Verfügung stehender Auswertelogiken ausgewählte Auswertelogik für die Ortsinformationen umfasst;
einen Vergleicher (56), der ausgebildet ist, um die Mehrzahl von Ortsinformationen mit dem vorbestimmten Übungsziel zu vergleichen; und
einen Bewerter (58), der ausgebildet ist, um den Verlauf der Trainingsübung basierend auf dem Ergebnis des Vergleiches zu bewerten, und
wobei das Positionsverfolgungssystem ferner ausgebildet ist, ein akustisches, visuelles oder haptisches Signal, das eine Information über eine Abweichung zwischen der mittels des Positionsverfolgungssystems bestimmten Position des Übungsobjektes innerhalb des Übungsareals und der Position der grafischen Repräsentation des Übungsobjektes (110a - 110j) innerhalb der grafischen Repräsentation des Übungsareals (100) umfasst, bereitzustellen, so dass die Position des Übungsobjektes innerhalb des Übungsareals beim Aufbau der Trainingsübung derart verändert werden kann, dass die mittels des Positionsverfolgungssystems bestimmte Position des Übungsobjektes innerhalb des Übungsareals der Position der grafischen Repräsentation des Übungsobjektes (110a -110j) innerhalb der grafischen Repräsentation des Übungsareals (100) entspricht.

9. Ein System (200) nach Anspruch 8, wobei das Positionserfassungssystem (200) ausgebildet ist, um die Ortsinformationen in Echtzeit zu erfassen und an den Analysator zu übermitteln.

10. Programm mit einem Programmcode, der bewirkt, dass das System gemäß Anspruch 8 oder Anspruch 9 das Verfahren nach einem der Ansprüche 1 bis 7 durchführt.

## Claims

1. A method for automatically evaluating a progression of a training exercise, comprising:
providing (10) a plurality of location information for at least one movable object (120; 120a, 120b) participating in the training exercise, wherein providing the plurality of location information comprises determining the position of the movable object (120; 120a, 120b) within an exercise area (100) using a position tracking system (200) at several successive points in time;
providing at least one further piece of location information using a graphical user interface which comprises a graphical representation of the exercise area (100) and a graphical representation of an exercise object (110a -110j) involved in the training exercise in a graphical display, wherein the further piece of location information indicates the position of the exercise object (110a - 110j) involved in the training exercise within the exercise area (100), and wherein the exercise object (110a - 110j) is generally movable, but moving the exercise object (110a - 110j) is not the objective of the training exercise;
providing (20) at least one predetermined exercise target, wherein providing (20) the exercise target comprises defining a desired interaction between the movable object (120) and the exercise object (110a - 110j) involved in the training exercise;
comparing (30) the plurality of location information to the exercise target; and
evaluating (40) the progression of the training exercise based on the result of the comparison (30), wherein providing (20) the at least one predetermined exercise target comprises selecting at least one used evaluation logic for the location information from a plurality of available evaluation logics,
wherein providing the further piece of location information comprises comparing the position of the graphical representation of the exercise object (110a -110j) within the graphical representation of the exercise area (100) to a position of the exercise object determined using the position tracking system within the exercise area, and
wherein the method further comprises providing an acoustic, visual or haptic signal by the position tracking system which comprises a piece of information about a deviation between the position of the exercise object determined using the position tracking system within the exercise area and the position of the graphical representation of the exercise object (110a - 110j) within the graphical representation of the exercise area (100), so that the position of the exercise object within the exercise area can be changed during set-up of the training exercise such that the position of the exercise object determined using the position tracking system within the exercise area corresponds to the position of the graphical representation of the exercise object (110a -110j) within the graphical representation of the exercise area (100).

2. The method of claim 1, wherein, according to the evaluation logic used, based on the location information a tactical parameter or a technical parameter is determined for a type of sport.

3. The method of claim 1, wherein, according to the evaluation logic used, based on the location information an athletic parameter is determined for at least one person involved in the training exercise.

4. The method of any of the preceding claims, wherein providing the further piece of location information comprises moving the graphical representation of the exercise object (110a -110j) on the graphical display.

5. The method of any of the preceding claims, wherein providing (20) the predetermined exercise target comprises defining parameters which are determined for the movable object (120) based on the location information to evaluate the progression of the training exercise.

6. The method of any of the preceding claims, wherein providing (20) the exercise target comprises defining all movable objects (120) and all exercise objects (110a - 110j) involved in the training exercise.

7. The method of any of the preceding claims, wherein evaluating the progression of the training exercise comprises displaying a visual illustration of the results of the comparison on a display.

8. A system for evaluating a progression of a training exercise, comprising:
a position detection system (200) configured to determine a position of a movable object (120; 120a, 120b) within an exercise area (100) at several successive points in time to provide a plurality of location information for the movable object (120a, 120b), and to determine a position of an exercise object within the exercise area (100), wherein the exercise object (110a - 110j) is generally movable, but moving the exercise object (110a - 110j) is not the objective of the training exercise; and
an analyzer (50) with an input interface (52) connected to the position detection system (200) for receiving the plurality of location information and the position of the exercise object determined by the position detection system (200),
wherein the analyzer further comprises the following:
a graphical user interface which comprises a graphical representation of the exercise area (100) and a graphical representation of the exercise object (110a -110j) in a graphical display, and by means of which a further piece of location information is provided, wherein the further piece of location information indicates the position of the exercise object (110a - 110i) involved in the training exercise within the exercise area (100);
a storage (54) configured to provide at least one predetermined exercise target, wherein the exercise target defines a desired interaction between the movable object (120) and the exercise object (110a - 110j) involved in the training exercise, wherein the at least one predetermined exercise target comprises at least one evaluation logic for the location information selected from a plurality of available evaluation logics;
a comparator (56) configured to compare the plurality of location information to the predetermined exercise target; and
an evaluator (58) configured to evaluate the progression of the training exercise based on the result of the comparison, and
wherein the position tracking system is further configured to provide an acoustic, visual or haptic signal which comprises a piece of information about a deviation between the position of the exercise object determined using the position tracking system within the exercise area and the position of the graphical representation of the exercise object (110a -110j) within the graphical representation of the exercise area (100), so that the position of the exercise object within the exercise area can be changed during set-up of the training exercise such that the position of the exercise object determined using the position tracking system within the exercise area corresponds to the position of the graphical representation of the exercise object (110a -110j) within the graphical representation of the exercise area (100).

9. A system (200) of claim 8, wherein the position detection system (200) is configured to detect the location information in real-time and to transmit the same to the analyzer.

10. A program with a program code which causes the system of claim 8 or claim 9 to perform the method of any of claims 1 to 7.

## Revendications

1. Procédé pour évaluer automatiquement un déroulement d'un exercice d'entraînement, comprenant :
une fourniture (10) d'une pluralité d'informations de localisation pour au moins un objet mobile (120 ; 120a, 120b) participant à l'exercice d'entraînement, dans lequel la fourniture de la pluralité d'informations de localisation comprend la détermination de la position de l'objet mobile (120 ; 120a, 120b) dans une zone d'exercice (100) au moyen d'un système de suivi de position (200) à plusieurs moments successifs ;
une fourniture d'au moins une information de localisation supplémentaire en utilisant une interface utilisateur graphique, qui comprend une représentation graphique de la zone d'exercice (100) et une représentation graphique d'un objet d'exercice (110a-110j) impliqué dans l'exercice d'entraînement dans une représentation graphique, dans lequel l'information de localisation supplémentaire indique la position du objet d'exercice (110a-110j) impliqué dans l'exercice d'entraînement dans la zone d'exercice (100), et dans lequel l'objet d'exercice (110a - 110j) est en principe mobile, une mouvement de l'objet d'exercice (110a - 110j) n'est toutefois pas le but de l'exercice d'entraînement ;
une fourniture (20) d'au moins un but d'exercice prédéterminé, dans lequel la fourniture (20) du but d'exercice comprend la définition d'une interaction recherchée entre l'objet mobile (120) et l'objet d'exercice (110a-110j) impliqué dans l'exercice d'entraînement ;
une comparaison (30) de la pluralité d'informations de localisation avec le but d'exercice ; et
une évaluation (40) du déroulement de l'exercice d'entraînement sur la base du résultat de la comparaison (30), dans lequel la fourniture (20) dudit au moins un but d'exercice prédéterminé comprend une sélection d'au moins une logique d'interprétation utilisée pour les informations de localisation d'une pluralité de logiques d'interprétation disponibles,
dans lequel la fourniture de l'information de localisation supplémentaire comprend une comparaison de la position de la représentation graphique de l'objet d'exercice (110a -110j) à l'intérieur de la représentation graphique de la zone d'exercice (100) à une position de l'objet d'exercice déterminée au moyen d'un système de suivi de position dans la zone d'exercice, et
dans lequel le procédé comprend en outre une fourniture d'un signal acoustique, visuel ou haptique par le système de suivi de position qui fournit une information sur un écart entre la position de l'objet d'exercice déterminée au moyen d'un système de suivi de position dans la zone d'exercice et la position de la représentation graphique de l'objet d'exercice (110a-110j) à l'intérieur de la représentation graphique de la zone d'exercice (100), de sorte que la position de l'objet d'exercice dans la zone d'exercice peut être modifiée dans le cadre de la mise en place de l'exercice d'entraînement, de sorte que la position de l'objet d'exercice déterminée au moyen d'un système de suivi de position dans la zone d'exercice correspond à la position de la représentation graphique de l'objet d'exercice (110a-110j) à l'intérieur de la représentation graphique de la zone d'exercice (100).

2. Procédé selon la revendication 1, dans lequel, selon la logique d'interprétation utilisée, sur la base des informations de localisation un paramètre tactique ou un paramètre technique est déterminé pour un type de sport.

3. Procédé selon la revendication 1, dans lequel, selon la logique d'interprétation utilisée, sur la base des informations de localisation, un paramètre athlétique est déterminé pour au moins une personne impliquée dans l'exercice d'entraînement.

4. Procédé selon l'une des revendications précédentes, dans lequel la fourniture de l'information de localisation supplémentaire comprend le déplacement de la représentation graphique de l'objet d'exercice (110a -110j) sur l'affichage graphique.

5. Procédé selon l'une des revendications précédentes, dans lequel la fourniture (20) du but d'exercice prédéterminé comprend une définition de paramètres qui sont déterminés pour l'objet mobile (120) sur la base des informations de localisation pour évaluer le déroulement de l'exercice d'entraînement.

6. Procédé selon l'une des revendications précédentes, dans lequel la fourniture (20) du but d'exercice comprend une définition de tous les objets mobiles (120) et de tous les objets d'exercice (110a - 110j) impliqués dans l'exercice d'entraînement.

7. Procédé selon l'une des revendications précédentes, dans lequel l'évaluation du déroulement de l'exercice d'entraînement comprend l'affichage d'une illustration visuelle des résultats de la comparaison sur un dispositif d'affichage.

8. Système pour évaluer un déroulement d'un exercice d'entraînement, comprenant :
un système de détection de position (200) configuré pour déterminer une position d'un objet mobile (120 ; 120a, 120b) dans une zone d'exercice (100) à plusieurs moments successifs pour fournir une pluralité d'informations de localisation pour l'objet mobile (120a, 120b), et pour déterminer une position d'un objet d'exercice dans la zone d'exercice (100), dans lequel l'objet d'exercice (110a-110j) est en principe mobile, une mouvement de l'objet d'exercice (110a - 110j) n'est toutefois pas le but de l'exercice d'entraînement ; et
un analyseur (50) avec une interface d'entrée (52) connectée avec le système de détection de position (200) pour recevoir la pluralité d'informations de localisation et la position de l'objet d'exercice déterminée par le système de détection de position,
dans lequel l'analyseur comprend en outre :
une interface utilisateur graphique qui comprend une représentation graphique de la zone d'exercice (100) et une représentation graphique de l'objet d'exercice (110a - 110j) dans un affichage graphique, et au moyen de laquelle une information de localisation supplémentaire est fournie, dans lequel l'information de localisation supplémentaire indique la position de l'objet d'exercice (110a - 110i) impliqué dans l'exercice d'entraînement dans la zone d'exercice (100) ;
un stockage (54) configuré pour fournir au moins un but d'exercice prédéterminé, dans lequel le but d'exercice définit une interaction recherchée entre l'objet mobile (120) et l'objet d'exercice (110a-110j) impliqué dans l'exercice d'entraînement, dans lequel ledit au moins un but d'exercice prédéterminé comprend au moins une logique d'interprétation sélectionnée d'une pluralité de logiques d'interprétation disponibles pour les informations de localisation ;
un comparateur (56) configuré pour comparer la pluralité d'informations de localisation au but d'exercice prédéterminé ;
un évaluateur (58) configuré pour évaluer le déroulement de l'exercice d'entraînement sur la base du résultat de la comparaison, et
dans lequel le système de suivi de position est en outre configuré pour fournir un signal acoustique, visuel ou haptique qui fournit une information sur un écart entre la position de l'objet d'exercice déterminée au moyen du système de suivi de position dans la zone d'exercice et la position de la représentation graphique de l'objet d'exercice (110a-110j) à l'intérieur de la représentation graphique de la zone d'exercice (100), de sorte que la position de l'objet d'exercice dans la zone d'exercice peut être modifiée dans le cadre de la mise en place de l'exercice d'entraînement, de sorte que la position de l'objet d'exercice déterminée au moyen d'un système de suivi de position dans la zone d'exercice correspond à la position de la représentation graphique de l'objet d'exercice (110a-110j) à l'intérieur de la représentation graphique de la zone d'exercice (100).

9. Système (200) selon la revendication 8, dans lequel le système de détection de position (200) est configuré pour enregistrer les informations de localisation en temps réel et pour les transmettre à l'analyseur.

10. Programme avec un code de programme qui provoque l'exécution du procédé selon l'une des revendications 1 à 7 par le système selon la revendication 8 ou la revendication 9.
